# FASCICULE DE BREVET EUROPEEN

(11) **EP 0 665 003 B1**
(45) Date de publication et mention de la délivrance du brevet: **28.08.1996**
(21) Numéro de dépôt: 94402979.2
(22) Date de dépôt: 21.12.1994
(51) Int. Cl.: A61K 7/00, A61K 7/42, A61K 7/48, A61K 49/00, A61K 9/127

(54) **Utilisation d'un accepteur de spin dans une composition cosmétique ou dermatologique**
Die Anwendung von Spinn-Akzeptor in einer kosmetischen oder dermatologischen Zusammensetzung
The use of a spin acceptor in a cosmetic or dermatological composition

(30) Priorité: 30.12.1993 FR 9315869
(43) Date de publication de la demande: 02.08.1995
(73) Titulaire: L'OREAL, F-75008 Paris (FR)
(72) Inventeur: Ribier, Alain, F-75001 Paris (FR); Nguyen, Quang Lan, F-92160 Antony (FR); Simonnet, Jean-Thierry, F-75011 Paris (FR); Boussouira, Boudiaf, F-75013 Paris (FR)
(74) Mandataire: Lhoste, Catherine

(56) Documents cités:
- EP-A- 0 433 131
- EP-A- 0 559 502
- WO-A-92/21017
- FR-A- 2 315 991
- FR-A- 2 408 387
- FR-A- 2 614 787
- US-A- 4 849 346
- US-A- 4 863 717
- INTERNATIONAL JOURNAL OF PHARMACEUTICS, vol. 62, ELSEVIER NL, page 75 V. GABRIJELCIC ET AL. 'Evaluation of liposomes as drug carriers into the skin by one-dimensional EPR imaging'
- PERIODICUM BIOLOGORUM, vol. 93, ZAGREB, page 245 V. GABRIJELCIC ET AL. 'liposome entrapped molecules penetration into the skin measured by nitroxide reduction kinetic imaging'

## Description

La présente invention se rapporte à l'utilisation d'un accepteur de spin dans une composition cosmétique et/ou dermatologique, pour le traitement photo-protecteur, antivieillissement et/ou antiacnéïque de la peau, y compris le cuir chevelu. L'invention se rapporte aussi à un procédé de traitement cosmétique de la peau par voie topique ainsi qu'à une composition cosmétique et/ou dermatologique contenant cet accepteur de spin.

Pour assurer une protection efficace de la peau contre les rayons du soleil, diminuer les effets de l'âge et supprimer les imperfections acnéïques de la peau, il est actuellement nécessaire d'utiliser plusieurs actifs antioxydants : à ce jour, un soin efficace de la peau contre ces effets et imperfections est obtenu en incorporant 4 à 8 actifs antioxydants. Or, l'introduction d'un grand nombre d'actifs dans une composition cosmétique et/ou dermatologique complique sa fabrication, entraînant en particulier une augmentation de son prix de revient.

L'invention a justement pour objet une composition cosmétique et/ou dermatologique ainsi que son utilisation pour le traitement photo-protecteur, antivieillissement et/ou antiacnéïque de la peau permettant de remédier à ces inconvénients.

De façon surprenante, le demandeur a trouvé qu'il était possible d'incoporer dans des compositions de traitement cosmétique et/ou dermatologique de la peau des accepteurs de spin ayant des propriétés photo-protectrices, antivieillissement, et/ou antiacnéïques.

Les accepteurs de spin et plus spécialement les radicaux nitroxydes libres sont généralement utilisés en spectroscopie comme sonde pour la résonance paramagnétique électronique (RPE), comme décrit dans le document "Electron paramagnetic resonance (EPR) imaging in skin : Biophysical and biochemical microscopy" de J. Fuchs et al., vol.98, n° 5, 1992 p. 713 à 719 et US-A-4849346. La mesure est basée sur la réduction de l'accepteur de spin. Les radicaux nitroxydes libres sont aussi utilisés pour visualiser des organes humains, par résonance magnétique.

De façon plus précise, l'invention a pour objet une utilisation cosmétique pour le traitement photo-protecteur, antivieillissement et/ou antiacnéïque de la peau, d'un accepteur de spin employé comme sonde de mesure en résonance paramagnétique électronique, dans une composition cosmétique ou dermatologique.

L'invention a aussi pour objet l'utilisation de cet accepteur spin pour la fabrication d'une composition dermatologique pour le traitement photo-protecteur antivieillissement et/ou antiacnéïque.

On a déjà envisagé d'injecter une solution de N-tert-butyl-α-phénylnitrone à des rongeurs en vue de diminuer le niveau de protéines oxydées et d'augmenter l'activité de la glutamine synthétase et de la protéase neutre dans leur cerveau : voir à ce sujet le document de J. M. Carney et al. Proc. Natl. Acad. Sci. vol.88, pp. 3633-3636 de mai 1991, "Reversal of age-related increase in enzyme activity, and loss in temporal and spatial memory by chronic administration of the spin-trapping compound N-tert-butyl-α-phenylnitrone" ; mais ce document n'envisage nullement l'emploi de cette nitrone dans une composition cosmétique et/ou dermatologique pour un traitement antivieillissement, antiacnéïque et/ou photo-protecteur de la peau.

Par ailleurs, il est connu du document EP-A-0327263 d'appliquer sur le cuir chevelu une composition contenant un précurseur de radicaux libres et un adjuvant tel qu'un agent réducteur, un antioxydant ou un séquestrant de radicaux hydroxyle afin de stimuler la pousse des cheveux. Cependant, ce document n'enseigne nullement l'emploi de ce précurseur de radicaux libres pour le traitement photo-protecteur, antivieillissement et/ou antiactéïque de la peau.

L'invention a aussi pour objet une composition cosmétique ou dermatologique pour traitement topique, telle que définie dans les revendications 24 et 36. Cette composition peut être appliqué aussi bien sur le visage et/ou le corps humain.

La composition de l'invention contient avantageusement un seul accepteur de spin, ce qui facilite sa fabrication et abaisse son coût de fabrication par rapport aux compositions actuellement connues pour le traitement photo-protecteur, antivieillissement et/ou antiacnéïque de la peau. En effet, les accepteurs de spin utilisés dans l'invention présentent l'avantage de pouvoir réagir avec toutes les espèces chimiques radicalaires hydrophiles de l'oxygène comme O₂·-, OH·, et les espèces radicalaires lipophiles comme CH₃ ; susceptibles de se former dans la peau alors que les actifs classiquement utilisés dans le domaine cosmétique comme anti-radicaux libres de l'oxygène (vitamine E, BHT - tertiobutylhydroxytoluène-, par exemple) sont spécifiques du type de radical ROO·, formé postérieurement à l'initiation des dégâts causés par les formes radicalaires hydrophiles sur les lipides des membranes cellulaires cutanées. Or, l'espèce OH· est la plus réactive vis-à-vis des biomolécules.

Personne jusqu'à ce jour n'avait fait le lien entre la capture des spins et la photoprotection, la lutte contre le vieillissement et/ou l'acné.

Les accepteurs de spin utilisables dans l'invention sont notamment les radicaux nitroxydes et les nitrones. En particulier, ces accepteurs de spin comportent un ou plusieurs cycles dont l'un au moins comporte un atome d'azote lié à l'atome d'oxygène.

Les accepteurs de spin de la composition de l'invention sont par exemple ceux cités dans le document Janssen Chimica Acta, vol.8,n°3 p.12-19(1990) de L.B.Volodarsky "Advances in the chemistry of stable nitroxides".

En particulier, l'accepteur de spin de l'invention est choisi parmi les radicaux 2,2,6,6-tétraméthylpipéridine-1-oxyle, 4-hydroxytétraméthyl-pipéridine-1-oxyle, N-tertbutyl-alpha-phénylnitrone, doxylcyclohexane, les sels de N-(1-oxyl-2,2,6,6-tétraméthyl-4-pipéridinyl)-N-diméthyl-N-hydroxyéthylammonium tel que l'ASL [l'iodure de N-(1-oxyl-2,2,6,6-tétraméthyl-4-pipéridinyl)-N-diméthyl-N-hydroxyéthylammonium] et leurs mélanges.

De façon avantageuse, l'accepteur de spin est présent à raison de 0,001% à 5% en poids par rapport au poids total de la composition, et mieux de 0,1% à 2% en poids.

Selon l'invention, la composition peut se présenter sous la forme d'un gel, d'une émulsion, d'un sérum, d'une lotion contenant avantageusement des sphérules de préférence lipidiques ou sous forme de gouttelettes d'huile dispersées par des vésicules lipidiques. L'emploi de sphérules non lipidiques est cependant possible ; dans ce cas, on peut utiliser des nanoparticules polymériques telles que les nanosphères et les nanocapsules.

On entend par sphérules ou vésicules lipidiques des particules formées d'une membrane constituée par un ou plusieurs feuillets concentriques, ces feuillets comportant une ou plusieurs couches bimoléculaires de lipides amphiphiles encapsulant une phase aqueuse. La phase aqueuse peut contenir des substances actives hydrosolubles et les couches bimoléculaires de lipides amphiphiles peuvent contenir des substances actives lipophiles. Ces sphérules ont généralement un diamètre moyen compris entre 10nm et 5000 nm.

Les sphérules lipidiques utilisées dans l'invention sont en particulier des sphérules aptes à libérer l'accepteur de spin dans les couches profondes de la peau, c'est-à-dire dans l'épiderme et le derme. En effet les sites de la peau les plus sensibles aux espèces radicalaires de l'oxygène se situent au niveau de l'épiderme et du derme. En particulier, les espèces radicalaires de l'oxygène provoquent des dégats importants notamment dans les membranes cellulaires (perméabilité des membranes), les noyaux des cellules (mutation par action sur l'ARN ou ADN), les tissus (nécroses, dégénérescence) entraînant un vieillissement cutané ; ces espèces réagissent sur les différents constituants de la peau.

Les sphérules aptes à diffuser dans les couches profondes de la peau, appelées aussi sphérules de profondeur, se trouvent en particulier à l'état gélifié à température ambiante (20 °C). En outre, ces sphérules présentent une constante de diffusion de l'ASL encapsulé dans le *stratum corneum*, constituant les couches superficielles de la peau >1.10⁻⁷ cm²s⁻¹.

La mesure de la constante de diffusion D s'effectue par combinaison de deux méthodes utilisant une sonde paramagnétique, l'ASL : la résonance paramagnétique électronique (RPE) unidimensionnelle et périodique d'une part et l'imagerie cinétique RPE d'autre part. Ces deux méthodes sont décrites respectivement dans les articles International Journal of Pharmaceutics, 62(1990) p. 75-79, Elsevier de V. Gabrijelcic et al. "Evaluation of liposomes as drug carriers into the skin by one-dimensional EPR imaging" et Periodicum Biologorum vol. 93, n° 2, p. 245-246, (1991) de V. Gabrijelcic et al. "Liposome entrapped molecules penetration into the skin measured by nitroxide reduction kinetic imaging".

Par ailleurs, ces sphérules de profondeur présentent un fort pouvoir d'encapsulation.

Le glucose est un marqueur classiquement utilisé pour ce type de détermination (cf. notamment Liposomes a practical approach de R.R.C. New, IRL Press (1990), p. 125-136). Le taux d'encapsulation est exprimé par le volume de solution de glucose, encapsulée dans les sphérules, mesuré en µl par rapport à l'unité de poids (mg) des lipides constituant la membrane. Ce taux d'encapsulation est déterminé immédiatement après l'étape de séparation du glucose libre et du glucose encapsulé (Tₒ) ainsi que vingt-quatre heures après cette séparation (T₂₄ heures).

La différence entre ces 2 déterminations successives illustre la perméabilité des sphérules vis-à-vis du glucose encapsulé.

Les sphérules (délivrant l'accepteur de spin dans les couches profondes de la peau) présentent un fort pouvoir d'encapsulation des petites molécules hydrosolubles classiquement modélisées par le glucose, ce pouvoir d'encapsulation se maintenant au moins 24 heures.

Les sphérules de profondeur peuvent être formées de lipides comportant au moins une chaîne grasse linéaire et saturée ayant de 16 à 30 atomes de carbone, tels que les phospholipides hydrogénés (de plantes ou d'oeuf), les phospholipides synthétiques saturés tels que la dipalmitoylphosphatidylcholine, les alkyléthers ou alkyesters de polyols à une, deux ou trois chaînes grasses par molécule. Ces lipides sont utilisés seuls ou en mélange.

L'invention a encore pour objet un procédé d'application par voie topique de la composition définie ci-dessus pour le traitement cosmétique antivieillissement, antiacnéïque et/ou photo-protecteur de la peau.

Selon l'invention, les accepteurs de spin ont la particularité de réagir avec les espèces chimiques de l'oxygène ayant une durée de vie très courte et donc une réactivité importante, c'est-à-dire avec les espèces hydrophiles de l'oxygène, empêchant, entre autre, la formation des radicaux ROO·. Ils permettent ainsi de protéger efficacement notamment les lipides, les protides et les acides nucléiques (ARN, ADN) de la peau.

Il est avantageusement possible d'associer à l'accepteur de spin, un filtre antisolaire afin d'empêcher la formation d'oxygène singulet dans la peau, lors de l'action du soleil sur la peau, et améliorer ainsi l'efficacité de la composition de l'invention dans un traitement antivieillissement, antiacnéïque et/ou photo-protecteur de la peau. Ce filtre peut consister en particulier en un filtre antirayonnement UVA et/ou UVB de nature organique hydrophile ou lipophile tel que l'acide benzène 1,4-di (3-méthylidène-10-camphosulfonique) ou de nature minérale tel que les nanopigments (TiO₂) ; il assure notament une protection de surface de la peau, complémentaire de la protection en profondeur.

Pour ce faire, le filtre est de préférence encapsulé dans des sphérules notamment lipidiques aptes à diffuser dans les couches superficielles de la peau. Ces sphérules, dites de surface, sont en particulier à l'état fluide à température ambiante (20 °C). Par ailleurs, elles assurent une diffusion de l'ASL dans le *stratum corneum* selon un coefficient de diffusion D <10⁻⁷ cm⁻²s⁻¹. De plus, elles présentent un faible pouvoir d'encapsulation. Autrement dit, ces sphérules retiennent le glucose encapsulé pendant moins de 24 heures.

Par ailleurs, les 2 catégories de sphérules peuvent contenir d'autres types d'actifs cosmétiques tels que les agents hydratants (polyols et plus particulièrement glycérine), kératolytiques (n-octanoyl -5 salicylique), anti-inflammatoires, antimicrobiens (triclosan), les vitamines (A, C, F), les protides (acides aminés, peptides, protéïnes), les sucres (fructose), les oligoéléments (Fe, Mg, Se), les enzymes (lipases, protéases, les enzymes de réparation de l'ADN), les huiles essentielles, etc... .

Les lipides principaux constituant les sphérules de surface sont choisis en particulier dans le groupe comprenant des lipides ioniques tels que notamment les phospholipides naturels à base de plantes ou d'oeuf, contenant des chaînes grasses insaturées ayant de 16 à 30 atomes de carbone ; des lipides non ioniques tels que notamment les alkyléthers ou alkylesters de polyols comportant une ou plusieurs chaînes grasses par molécule, dont au moins une chaîne grasse de longueur inférieure à 16 atomes de carbone, tels que dans le lauryl polyglycéryl-6-cétéarylglycoléther ainsi que leurs mélanges. Ce dernier éther est décrit en détail dans la demande de brevet FR 92-09603 déposée par L'Oréal.

On peut de façon connue incorporer dans la phase lipidique constituant la membrane lipidique des sphérules au moins un additif choisi dans le groupe formé des stérols (phytostérols, cholestérol, phytostérols polyoxyéthylénés), des alcools, diols et triols à longue chaîne (phytanetriol), des amines à longue chaîne et leurs dérivés ammonium quaternaire et/ou un additif ionique choisi dans le groupe formé des esters phosphoriques d'alcools gras et leurs sels alcalins (Na, K) tels que le dicétylphosphate, le dicétylphosphate de Na, les alkylsulfates (cétylsulfate de sodium), les sels alcalins du cholestérol sulfate ou du cholestérol phosphate, le sel de sodium de l'acide phosphatidique, les lipoaminoacides et leurs sels tels que les acylglutamates de sodium.

La composition de l'invention peut, entre outre, contenir tous les composants classiquement utilisés dans le domaine cosmétique et en particulier une huile végétale, minérale, siliconée, synthétique dispersée dans une phase aqueuse et également des adjuvants hydrophiles comme les gélifiants, les conservateurs, les opacifiants, des adjuvants lipophiles tels que les parfums, des pigments et des charges comme décrit dans les documents FR-A-2490504 et FR-A-2485921. L'huile dispersée peut représenter de 2 % à 40 % en poids par rapport au poids total de la composition et les adjuvants peuvent représenter de 0,1 % à 10 % en poids.

On peut citer comme exemple de vésicules lipidiques (vésicules de première catégorie) délivrant l'accepteur de spin dans les couches profondes de la peau, les vésicules obtenues à partir des lipides suivants (nom CTFA) :
- A / cholestérol / lipoaminoacide caséique notamment dans un rapport pondéral 45/45/10 (où A est un cétyléther de triglycéryle commercialisé par la société Chimex sous le nom Chimexane NL) ;
- B / cholestérol / dicétylphosphate notamment dans un rapport pondéral 60/35/5 (où B est un mélange de mono-, di- et tri-cétyléther de triglycéryle commercialisé par la société Chimex sous le nom Chimexane NT) ;
- Span 40 (de chez Ici ou palmitate de sorbitane) / cholestérol / acylglutamate de sodium (commercialisé sous le nom HS11 par la société Ajinomoto) notamment dans un rapport pondéral 47,5/47,5/5 ;
- Stéarate de PEG 8 / cholestérol / acylglutamate de sodium avec notamment un rapport pondéral 47,5/47,5/5 (où le stéarate de PEG 8 est le polyéthylène glycol à 8 motifs d'oxyde d'éthylène commercialisé par Unichema sous le nom Stéarate de PEG 400) ;
- Stéarate de PEG 8 / cholestérol / phytanetriol / acylglutamate de sodium avec notamment un rapport pondéral 47,5/20/27,5/5 ;
- Lécithine hydrogénée / phytostérol polyoxyéthyléné à 5 motifs d'oxyde d'éthylène notamment dans un rapport pondéral 60/40 ;
- Distéarate de méthylglucose polyoxyéthyléné à 20 motifs d'oxyde d'éthylène / cholestérol / acylglutamate de sodium notamment dans un rapport pondéral 45/45/10 (le distéarate est par exemple commercialisé par Amerchol sous le nom Glucam E 20 distearate) ;
- A / cholestérol / dicétylphosphate avec notamment un rapport pondéral 47,5/47,5/5 ;
- Distéarate de diglycéryle (par exemple Emalex DS G2 commercialisé par Nihon) / cholestérol / acylglutamate de sodium dans un rapport pondéral 45/45/10 ;
- Mono- et di-stéarate de saccharose ( par exemple Grilloten PSE 141 G de chez Grillo) / cholestérol / acylglutamate de sodium notamment dans un rapport pondéral 45/45/10 ;
- Tristéarate de tétraglycéryle (par exemple Tetraglyn 3S de chez Nikkol) / cholestérol / acylglutamate de sodium notamment dans un rapport pondéral de 45/45/10.

On peut citer comme exemples de vésicules (de deuxième catégorie) délivrant un actif de surface (par exemple un filtre, un agent hydratant) dans les couches superficielles de la peau, les vésicules obtenues à partir des lipides suivants :
- Lécithine de tournesol ;
- Natipide II (lécithine de soja / éthanol / eau dans un rapport pondéral 60/20/20 commercialisé par Nattermann) ;
- C (lécithine de soja / cholestérol / propylène glycol dans un rapport pondéral 40/30/30 commercialisé par Nattermann sous le nom NAT 50 PG) ;
- D / dimyristylphosphate notamment dans un rapport pondéral 95/5 (où D est un éther de lauryl polyglycéryl-6-cétéarylglycol commercialisé par Chimex sous le nom Chimexane NS).

Le tableau I ci-après donne, pour certaines vésicules lipidiques obtenues à partir des lipides ci-dessus, la constante de diffusion D de l'ASL dans le *Stratum Corneum* et dans l'épiderme/derme ainsi que le taux d'encapsulation du glucose, et la température de transition de phase du lipide principal constituant la membrane. La constante de diffusion a été mesurée pour une concentration en ASL encapsulé de 0,35 % en poids par rapport au poids total de la composition.

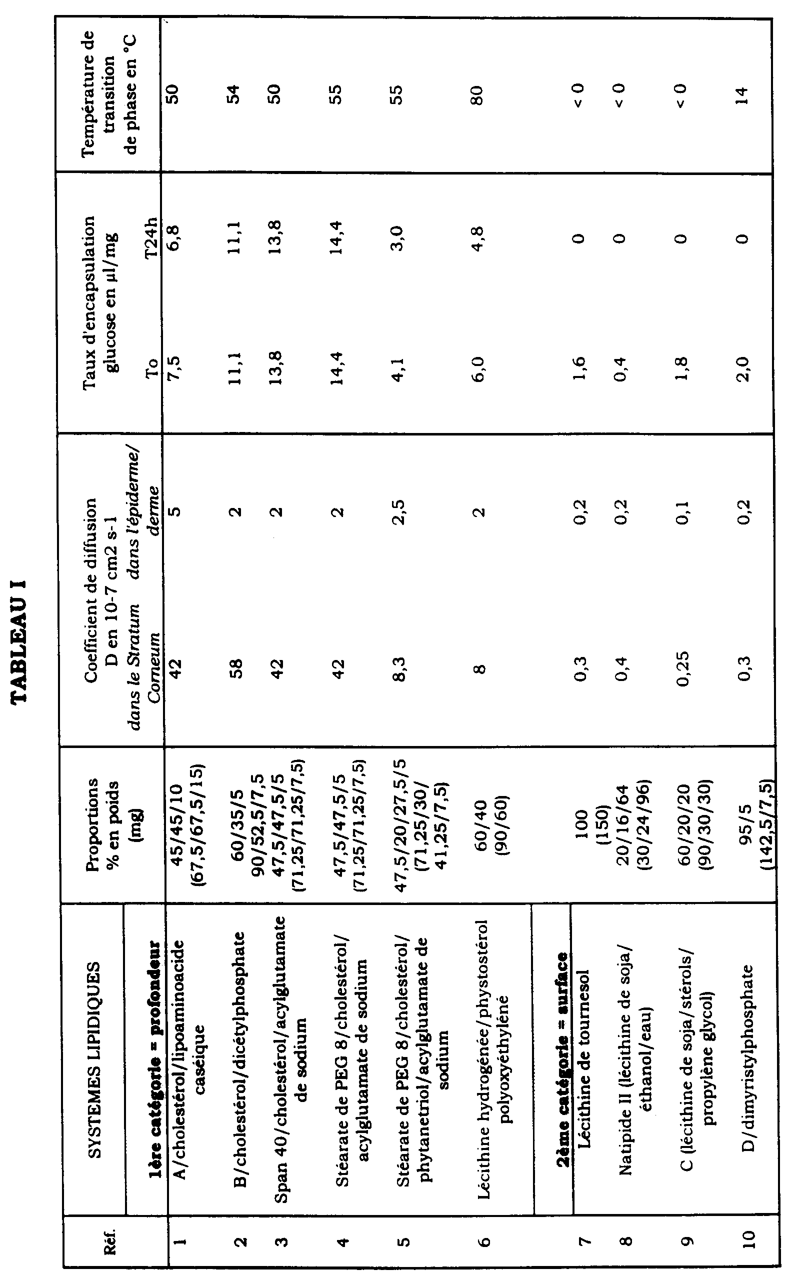

La mesure du taux d'encapsulation s'effectue comme décrit dans le document RRC New cité ci-dessus et celle de la constante de diffusion D comme décrit précédemment.

Pour reconnaitre l'état des vésicules, on détermine la température de transition de phase (lamellaire fluide-gel) du lipide principal constituant leur membrane, par analyse thermique différentielle (ATD).

L'invention a encore pour objet un procédé pour le traitement photo-protecteur, antivieillissement et/ou antiacnéïque de la peau, consistant à appliquer sur la peau la composition définie ci-dessus.

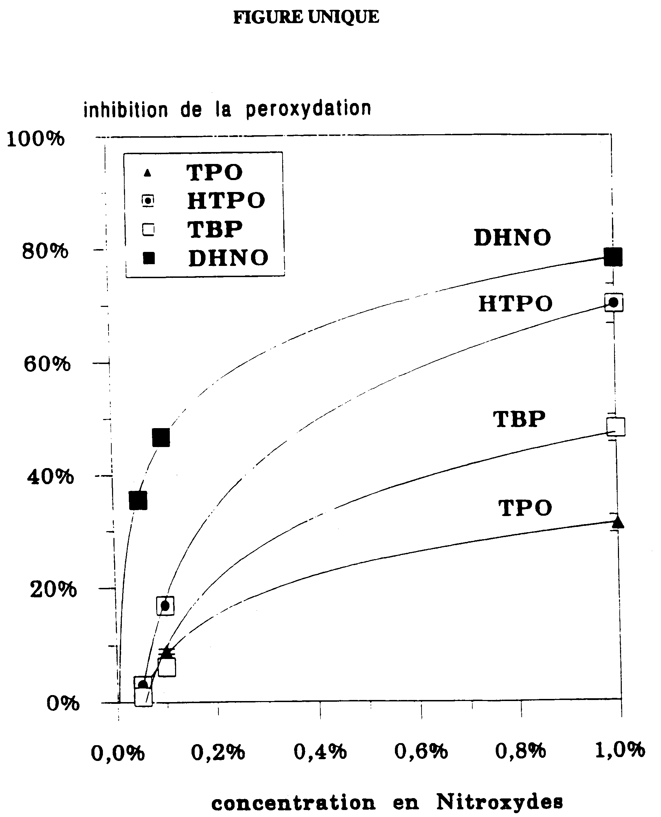

D'autres caractéristiques et avantages de l'invention ressortiront mieux de la description qui suit, donnée à titre illustratif et non limitatif en référence à l'unique figure annexée qui représente des courbes montrant l'inhibition de la peroxydation du sébum (liquide lipidique produit à la surface de la peau) par des radicaux nitroxydes. Ces tests ont été réalisés avec des compositions simples contenant de 0,05% à 1% en poids de radicaux nitroxydes par rapport au poids de la composition, le reste de la composition étant constitué d'eau. Ces tests ont été réalisés ex-vivo en mettant les différentes compositions au contact de sébum puis en les irradiant par des UVA à une dose de 5 joules par cm² d'échantillon.

Les radicaux testés sont le 2,2,6,6-tétraméthylpipéridine-1-oxyle (TPO), le 4-hydroxytétraméthyl-pipéridine-1-oxyle (HTPO), le N-tertbutyl-alpha-phénylnitrone (TBP), le doxylcyclohexane (DHNO), et correspondent respectivement aux courbes a, b, c, d de la figure.

De ces courbes, on constate une faible inhibition de la péroxydation du sébum dans la gamme de concentrations comprise entre 0% et 0,1% pour le TPO, le HPTO, et le TBP. Cette inhibition est voisine de celle obtenue avec le BHT à la même concentration. Dans le cas du DHNO, on observe une inhibition qui est supérieure à celle du BHT.

Cette inhibition devient plus important pour 1% d'actif. Elle est maximale pour le DHNO et le HTPO, respectivement 78% et 75% d'inhibition contre 30% pour TPO et 50% pour le TBP.

En conséquence ces quatre nitroxydes radicalaires présentent une certaine efficacité contre la péroxydation du sébum.

On décrit ci-après la fabrication de compositions à base de vésicules lipidiques de surface et de profondeur, conformes à l'invention, utilisant comme actif de profondeur un radical nitroxyde.

### A) Obtention de vésicules lipidiques renfermant l'ASL

Les lipides constitutifs de la paroi des vésicules sont pesés et dissous dans 10 ml de méthanol. La solution alcoolique est alors transvasée dans un flacon rond de 50 ml à col rodé, que l'on place ensuite sur un évaporateur rotatif, de telle façon que le contenu soit thermostaté à la température de 30 °C. L'évaporation est poussée jusqu'au dépôt d'un film sec de lipides sur les parois du flacon.

3 ml d'une solution aqueuse 0,01 molaire d'ASL sont alors ajoutés dans le ballon qui est ensuite secoué à la main pendant environ 10 minutes, soit à la température ambiante (20 °C) pour les vésicules du tableau I référencées 7 à 10, soit à la température de 50 °C pour les vésicules référencées 1 à 6 du tableau I. On laisse alors le milieu s'équilibrer à température ambiante, pendant 2 heures, puis on place la dispersion dans un sac de dialyse et au contact de 500 ml d'eau distillée. La dialyse s'effectue pendant une nuit. Après une nuit, l'eau est changée et la dialyse est poursuivie pendant 4 heures supplémentaires.

Un fil de coton de 0,3 mm d'épaisseur est alors mis à tremper dans la dispersion de vésicules puis mis au contact d'une coupe de peau provenant d'une oreille de porc fraîchement récupérée dans un abattoir destiné à l'approvisionnement alimentaire.

L'échantillon d'oreille prélevé est rincé à l'eau et découpé en tranches de 1 mm d'épaisseur, 5 mm de large et 10 mm de long puis placé dans une cellule de maintien. Les mesures de diffusion de l'ASL dans la peau sont effectuées dans les 24 heures suivant le prélèvement de peau.

### B) Obtention de la composition cosmétique

### 1° Obtention des vésicules de première catégorie (diffusant en profondeur)

On prépare les vésicules (de profondeur) selon une méthode usuelle de co-fusion des constituants (voir tableau I) de la membrane choisis. Ainsi, on fond le constituant membranaire ayant le point de fusion T_{f} le moins élevé ; on ajoute les autres constituants membranaires puis on homogénéise sous agitation moyenne et enfin on hydrate partiellement en maintenant la température de fusion T_{f}, définie ci-dessus.

A la pâte obtenue, on ajoute une solution aqueuse d'un accepteur de spin pour le traitement en profondeur. Une turbine est actionnée pendant 1 h 30 pour bien hydrater, en maintenant la température T_{f}. On ajoute éventuellement au milieu réactionnel un ou plusieurs autres types d'actifs pour le traitement en profondeur (par exemple protides, sucres, oligoéléments, huiles essentielles), on homogénéise et diminue la température du milieu jusqu'à la température ambiante (20 °C).

### 2° Obtention de vésicules de seconde catégorie (diffusant en surface)

On introduit à température ambiante (20 °C) et par simple agitation une solution aqueuse d'un filtre ou plusieurs autres actifs (par exemple polyols, acides aminés, sucres, agents kératolytiques, vitamines) pour le traitement en surface dans le mélange choisi de constituants devant former la membrane des vésicules de surface (voir tableau I). On obtient ainsi des vésicules de surface encapsulant le filtre ou tout autre actif de surface.

### 3° Obtention de la composition "double liposomes"

Au milieu contenant les vésicules de profondeur, on ajoute la phase grasse (huiles) de la composition et on la disperse (à température ambiante) sous agitation. On mélange alors le milieu réactionnel obtenu à celui contenant les vésicules de surface. Eventuellement, on ajoute alors les adjuvants tels que des conservateurs, un gélifiant que l'on peut neutraliser si nécessaire par une base (triéthanolamine ou soude) et des parfums, etc...

Le produit obtenu se présente sous forme d'une crème blanche, douce et onctueuse utilisable dans le domaine cosmétique et/ou dermatologique pour protéger la peau des rayonnements lumineux (visible, UV), et lutter contre le vieillissement de la peau et/ou l'acné, en surface et en profondeur. Cette crème peut être utilisée tous les jours.

On donne ci-après des exemples particuliers de compositions cosmétiques conformes à l'invention. Les quantités sont données en pourcentage pondéral.

### EXEMPLE 1 : Crème double liposomes, photo-protectrice

| - *Liposomes de profondeur :* | |
|---|---|
| Cétyléther de triglycéryle / cholestérol / dicétylphosphate dans un rapportpondéral 47,5/47,5/5 | 3,0 % |
| TPO (actif) | 1,0 % |
| Eau déminéralisée | 15,0 % |

| - *Liposomes de surface :* | |
|---|---|
| Lécithine de soja | 3,0 % |
| Acide benzène 1,4-di(3-méthylidène-10-camphosulfonique)(filtre) | 0,5 % |
| Triéthanolamine (neutralisant) | 0,3 % |
| Glycérine (actif) | 3,0 % |
| Eau déminéralisée | 15,0 % |

| - *Phase aqueuse :* | |
|---|---|
| Polymère carboxyvinylique (gélifiant) | 0,4 % |
| Conservateurs | 0,3 % |
| Triéthanolamine | qsp pH=6 |
| Eau déminéralisée | qsp 100 % |

| - *Phase grasse :* | |
|---|---|
| Huiles végétales ou minérales | 10,0 % |
| Huile de silicone volatile | 5,0 % |

### EXEMPLE 2 : Crème double liposomes, dépigmentante

Cette crème se différencie de celle de l'exemple 1 par l'emploi de TBP au lieu de TPO comme actif de profondeur.

### EXEMPLE 3 : Crème double liposomes, antivieillissement

| - *Vésicules de profondeur :* | |
|---|---|
| Stéarate de PEG 8/cholestérol/acylglutamate de sodium dans un rapport pondéral 47,5/47,5/5 | 5,0 % |
| HTPO (actif membranaire) | 1,0 % |
| Eau déminéralisée | 15,0 % |

| - *Phase grasse :* | |
|---|---|
| Huile végétale | 10,0 % |
| Huile de silicone volatile | 5,0 % |

| - *Phase aqueuse :* | |
|---|---|
| Conservateurs | 0,3 % |
| Glycérine (actif) | 3,0 % |
| Polymère carboxyvinylique (gélifiant) | 0,4 % |
| Triéthanolamine | qsp pH=6 |
| Eau | qsp 100 % |

### EXEMPLE 4 : Crème double liposomes, antiacnéïque

| - *Vésicules de profondeur :* | |
|---|---|
| Palmitate de sorbitane/cholestérol/acylglutamate de sodium dans un rapport pondéral 47,5/47,5/5 | 4,0 % |
| DHNO (actif hydrosoluble) | 0,2 % |
| Eau déminéralisée | 15,0 % |

| - *Vésicules de surface :* | |
|---|---|
| Chimexane NS/Dimyristylphosphate dans un rapport pondéral 95/5 | 3 % |
| Octylméthoxycinnamate commercialisé sous le nom de Parsol MCX par la société Givaudan | 0,3 % |
| Glycérol (actif) | 3,0 % |
| Eau déminéralisée | 15,0 % |

| - *Phase grasse :* | |
|---|---|
| Huile végétale | 3,0 % |
| Huile de silicone volatile | 4,5 % |

| - *Phase aqueuse :* | |
|---|---|
| Triclosan | 0,2 % |
| Conservateurs | 0,3 % |
| Polymère carboxyvinylique (gélifiant) | 0,9 % |
| Soude | qsp pH=6 |
| Eau déminéralisée | qsp 100 % |

## Revendications

1. Utilisation cosmétique pour le traitement photo-protecteur, anti-vieillissement et/ou antiacnéïque de la peau, d'un accepteur de spin employé comme sonde de mesure en résonance paramagnétique électronique, dans une composition cosmétique ou dermatologique.

2. Utilisation d'un accepteur de spin employé comme sonde de mesure en résonance paramagnétique électronique pour la fabrication d'une composition dermatologique pour le traitement photo-protecteur, anti-vieillissement et/ou antiacnéïque de la peau.

3. Utilisation selon la revendication 1 ou 2, caractérisée en ce que l'accepteur de spin est un accepteur choisi parmi les radicaux nitroxydes et les nitrones cycliques stables comportant au moins un cycle contenant un atome d'azote lié à l'atome d'oxygène.

4. Utilisation selon la revendication 3, caractérisée en ce que l'accepteur de spin comporte des groupements alkyles en position α de l'atome d'azote.

5. Utilisation selon l'une quelconque des revendications précédentes, caractérisée en ce que l'accepteur de spin est choisi parmi les radicaux 2,2,6,6 tétraméthylpipéridine-1-oxyle, 4-hydroxy-tétraméthyl-pipéridine-1-oxyle,N-tertbutyl-alpha-phénylnitrone, doxylcyclohéxane, les sels de N-(1-oxyl-2,2,6,6-tétraméthyl-4-pipéridinyl)-N-diméthyl-N-hydroxyéthylanimonium.

6. Utilisation selon l'une quelconque des revendications précédentes, caractérisée en ce que l'accepteur de spin est présent à raison de 0,001% à 5% en poids par rapport au poids total de la composition.

7. Utilisation selon l'une quelconque des revendications précédentes, caractérisée en ce que l'accepteur de spin est présent à raison de 0,1% à 2% en poids par rapport au poids total de la composition.

8. Utilisation selon l'une quelconque des revendications précédentes, caractérisée en ce que l'accepteur de spin est encapsulé dans des sphérules.

9. Utilisation selon la revendication 8, caractérisée en ce que les sphérules sont des vésicules lipidiques.

10. Utilisation selon la revendication 8 ou 9, caractérisée en ce que les sphérules comportent des sphérules de profondeur aptes à diffuser dans les couches profondes de la peau.

11. Utilisation selon la revendication 10, caractérisée en ce que les sphérules de profondeur sont à l'état gélifié à la température ambiante.

12. Utilisation selon la revendication 10 ou 11, caractérisée en ce que les sphérules de profondeur sont formées de lipides comportant au moins une chaîne grasse linéaire et saturée ayant de 16 à 30 atomes de carbone.

13. Utilisation selon l'une quelconque des revendications 10 à 12, caractérisée en ce que les sphérules de profondeur sont formées par les lipides choisis parmi :
Cétyléther de triglycéryle/cholestérol/lipoaminoacide caséique ;
Mélange de mono-, di- et tri-cétyléther de triglycéryle/cholestérol/dicétylphosphate ;
Cétyléther de triglycéryle/cholestérol/dicétylphosphate ;
Palmitate de sorbitane/cholestérol/acylglutamate de sodium ;
Stéarate de PEG 8/cholestérol/acylglutamate de sodium ;
Distéarate de diglycéryle/cholestérol/acyglutamate de sodium ;
Mono et distéarate de saccharose/cholestérol/acylglutamate de sodium ;
Stéarate de PEG 8/cholestérol/phytanetriol/acylglutamate de sodium ;
Distéarate de méthylglucose polyoxyéthyléné à 20 moles d'oxyde d'éthyléne/cholestérol/acylglutamate de sodium ;
Lécithine hydrogénée/phytostérol polyoxyéthyléné ;
Tristéarate de tétraglycéryle/cholestérol/acylglutamate de sodium.

14. Utilisation selon l'une quelconque des revendications précédentes, caractérisée en ce qu'elle se présente sous la forme d'un gel, d'une émulsion, d'une lotion, d'un sérum, de gouttelettes d'huile dispersées par des vésicules lipidiques.

15. Utilisation selon l'une quelconque des revendications précédentes, caractérisée en ce qu'elle contient, en outre, une phase huileuse dispersée dans une phase aqueuse.

16. Utilisation selon l'une quelconque des revendications précédentes, caractérisée en ce qu'elle contient, en outre, des adjuvants hydrophiles.

17. Utilisation selon l'une quelconque des revendications précédentes, caractérisée en ce qu'elle contient un filtre antisolaire.

18. Utilisation selon la revendication 17, caractérisée en ce que le filtre antisolaire est encapsulé dans des sphérules de surface aptes à diffuser dans les couches superficielles de la peau.

19. Utilisation selon la revendication 18, caractérisée en ce que les sphérules de surface sont formées de lipides choisis parmi les phospholipides ioniques naturels contenant des chaînes grasses insaturées ayant de 16 à 30 atomes de carbone, les alkyléthers ou alkylesters de polyols ayant au moins une chaîne grasse par molécule dont au moins une chaîne grasse de longueur inférieure à 16 atomes de carbone, et leurs mélanges.

20. Utilisation selon la revendication 18 ou 19, caractérisée en ce que les sphérules de surface sont formées d'au moins un lipide choisi parmi :
Lécithine de tournesol ;
Lécithine de soja/éthanol/eau;
Lécithine de soja/cholestérol/propylène glycol ;
Ether de lauryl polyglycéryl-6-cétéarylglycol/dimyristylphosphate.

21. Composition cosmétique ou dermatologique pour traitement topique, caractérisée en ce qu'elle comprend une première dispersion de sphérules de profondeur aptes à diffuser dans les couches profondes de la peau, ces sphérules de profondeur contenant un accepteur de spin employé comme sonde de mesure en résonance paramagnétique électronique et étant aptes à libérer l'accepteur de spin dans les couches profondes de la peau, une seconde dispersion de sphérules de surface aptes à diffuser dans les couches superficielles de la peau, ces sphérules de surface contenant un filtre antisolaire et étant aptes à libérer le filtre antisolaire dans les couches superficielles de la peau pour assurer une protection de surface, complémentaire de la protection en profondeur et un milieu cosmétiquement ou dermatologiquement acceptable.

22. Composition selon la revendication 20, caractérisée en ce que l'accepteur de spin est choisi parmi les radicaux 2,2,6,6 tétraméthylpipéridine-1-oxyle, 4-hydroxytétraméthylpipéridine-1-oxyle, N -tertbutyl-alpha-phénylnitrone, doxylcyclohéxane, les sels de N-(1-oxyl-2,2,6,6-tétraméthyl-4-pipéridinyl)-N-diméthyl-N-hydroxyéthylammonium.

23. Composition selon l'une quelconque des revendications 21 à 22, caractérisée en ce que l'accepteur de spin est présent à raison de 0,001% à 5% en poids par rapport au poids total de la composition.

24. Composition selon l'une quelconque des revendications 21 à 23, caractérisée en ce que l'accepteur de spin est présent à raison de 0,1% à 2% en poids par rapport au poids total de la composition.

25. Composition selon l'une quelconque des revendications 21 à 24, caractérisée en ce que les sphérules de profondeur sont à l'état gélifié à la température ambiante.

26. Composition selon l'une quelconque des revendications 21 à 25, les sphérules de profondeur sont formées de lipides comportant au moins une chaîne grasse linéaire et saturée ayant de 16 à 30 atomes de carbone.

27. Composition selon l'une quelconque des revendications 21 à 26, caractérisée en ce que les sphérules de profondeur sont formées par les lipides choisis parmi :
Cétyléther de triglycéryle/cholestérol/lipoaminoacide caséique;
Mélange de mono-, di- et tri-cétyléther de triglycéryle/cholestérol/dicétylphosphate ;
Cétyléther de triglycéryle/cholestérol/dicétylphosphate ;
Palmitate de sorbitane/cholestérol/acylglutamate de sodium ;
Stéarate de PEG 8/cholestérol/acylglutamate de sodium ;
Distéarate de diglycéryle/cholestérol/acyglutamate de sodium ;
Mono et distéarate de saccharose/cholestérol/acylglutamate de sodium ;
Stéarate de PEG 8/cholestérol/phytanetriol/acylglutamate de sodium ;
Distéarate de méthylglucose polyoxyéthyléné à 20 moles d'oxyde d'éthylène/cholestérol/acylglutamate de sodium ;
Lécithine hydrogénée/phytostérol polyoxyéthyléné;
Tristéarate de tétraglycéryle/cholestérol/acylglutamate de sodium.

28. Composition selon l'une quelconque des revendications 21 à 27, caractérisée en ce que les sphérules de surface sont formées de lipides choisis parmi les phospholipides ioniques naturels contenant des chaînes grasses insaturées ayant de 16 à 30 atomes de carbone, les alkyléthers ou alkylesters à au moins une chaîne grasse par molécule dont au moins une chaîne grasse de longueur inférieure à 16 atomes de carbone, et leurs mélanges.

29. Composition selon l'une quelconque des revendications 21 à 28, caractérisée en ce que les sphérules de surface sont formées d'au moins un lipide choisi parmi :
Lécithine de tournesol ;
Lécithine de soja/éthanol/eau ;
Lécithine de soja/cholestérol/propylène glycol ;
Ether de lauryl polyglycéryl-6-cétéarylglycol/dimyristylphosphate.

30. Composition selon l'une quelconque des revendications 21 à 29, caractérisée en ce qu'elle se présente sous la forme d'un gel, d'une émulsion, d'une lotion, d'un sérum, de gouttelettes d'huile dispersées par des vésicules lipidiques.

31. Composition selon l'une quelconque des revendications 21 à 30, caractérisée en ce qu'elle contient, en outre, une phase huileuse dispersée dans une phase aqueuse.

32. Composition selon l'une quelconque des revendications 21 à 31, caractérisée en ce qu'elle contient, en outre, des adjuvants hydrophiles.

33. Composition selon l'une quelconque des revendications 21 à 32, caractérisée en ce que les sphérules contiennent, en outre, des actifs choisis parmi, les agents hydratants, kératolytiques, antimicrobiens et anti-inflammatoire, les vitamines, les protides, les sucres, les oligoéléments, les enzymes, les huiles essentielles.

34. Procédé de traitement cosmétique photo-protecteur, anti-vieillissement et/ou antiacnéïque de la peau, consistant à appliquer sur la peau une composition selon l'une quelconque des revendications 21 à 33.

35. Procédé de traitement cosmétique photo-protecteur, anti-vieillissement et/ou antiacnéïque, consistant à appliquer sur la peau du visage ou du corps humain une composition cosmétique ou dermatologique comprenant une première dispersion de sphérules de profondeur aptes à diffuser dans les couches profondes de la peau, ces sphérules contenant un accepteur de spin employé comme sonde de mesure en résonance paramagnétique électronique, et un milieu cosmétiquement ou dermatologiquement acceptable.

36. Composition cosmétique ou dermatologique comprenant une première dispersion de sphérules de profondeur aptes à diffuser dans les couches profondes de la peau, ces sphérules contenant un accepteur de spin employé comme sonde de mesure en résonance paramagnétique électronique, et un milieu cosmétiquement ou dermatologiquement acceptable, cette composition se présentant sous forme d'émulsion ou de gouttelettes d'huile dispersées par des vésicules lipidiques.

## Claims

1. Cosmetic use for the light-protective, anti-ageing and/or anti-acne treatment of the skin, of a spin trap employed as an electron paramagnetic resonance measurement probe, in a cosmetic or dermatological composition.

2. Use of a spin trap employed as an electron paramagnetic resonance measurement probe for the manufacture of a dermatological composition for the light-protective, anti-ageing and/or anti-acne treatment of the skin.

3. Use according to Claim 1 or 2, characterized in that the spin trap is a trap chosen from stable cyclic nitroxide and nitrone radicals comprising at least one ring containing a nitrogen atom which is bonded to the oxygen atom.

4. Use according to Claim 3, characterized in that the spin trap comprises alkyl groups in the position α to the nitrogen atom.

5. Use according to any one of the preceding claims, characterized in that the spin trap is chosen from the 2,2,6,6-tetramethylpiperidine 1-oxide, 4-hydroxytetramethylpiperidine 1-oxide, N-tert-butyl-alpha-phenylnitrone and doxylcyclohexane radicals, and the salts of N-(1-oxido-2,2,6,6-tetramethyl-4-piperidyl)-N,N-dimethyl-N-hydroxyethylammonium.

6. Use according to any one of the preceding claims, characterized in that the spin trap is present in an amount of from 0.001 % to 5 % by weight relative to the total weight of the composition.

7. Use according to any one of the preceding claims, characterized in that the spin trap is present in an amount of from 0.1 % to 2 % by weight relative to the total weight of the composition.

8. Use according to any one of the proceding claims, characterized in that the spin trap is encapsulated in spherules.

9. Use according to Claim 8, characterized in that the spherules are lipid vesicles.

10. Use according to Claim 8 or 9, characterized in that the spherules comprise spherules with deep-down action which are capable of diffusing into the deep layers of the skin.

11. Use according to Claim 10, characterized in that the spherules with deep-down action are in the gelled state at room temperature.

12. Use according to Claim 10 or 11, characterized in that the spherules with deep-down action are formed of lipids comprising at least one linear and saturated fatty chain having from 16 to 30 carbon atoms.

13. Use according to any one of Claims 10 to 12, characterized in that the spherules with deep-down action are formed by lipids chosen from;
Triglyceryl cetyl ether/cholesterol/casein lipoamino acid;
Mixture of triglyceryl mono-, di- and tricetyl ether/cholesterol/dicetyl phosphate;
Triglyceryl cetyl ether/cholesterol/dicetyl phosphate;
Sorbitan palmitate/cholesterol/sodium acylglutamate;
PEG 8 stearate/cholesterol/sodium acylglutamate;
Diglyceryl distearate/cholesterol/sodium acylglutamate;
Sucrose mono- and distearate/cholesterol/sodium acylglutamate;
PEG 8 stearate/cholesterol/phytanetriol/sodium acylglutamate;
Polyoxyethylenated methylglucose distearate containing 20 mol of ethylene oxide/cholesterol/sodium acylglutamate;
Hydrogenated lecithin/polyoxyethylenated phytosterol;
Tetraglyceryl tristearate/cholesterol/sodium acylglutamate.

14. Use according to any one of the preceding claims, characterized in that it is provided in the form of a gel, an emulsion, a lotion, a serum or oil droplets dispersed by lipid vesicles.

15. Use according to any one of the preceding claims, characterized in that it additionally contains an oily phase which is dispersed in an aqueous phase.

16. Use according to any one of the preceding claims, characterized in that it additionally contains hydrophilic adjuvants.

17. Use according to any one of the preceding claims, characterized in that it contains a sunscreen agent.

18. Use according to Claim 17, characterized in that the sunscreen agent is encapsulated in spherules active at the surface which are capable of diffusing into the surface layers of the skin.

19. Use according to Claim 18, characterized in that the spherules active at the surface are formed of lipids chosen from natural ionic phospholipids containing unsaturated fatty chains having from 16 to 30 carbon atoms, polyol alkyl ethers or polyol alkyl esters having at least one fatty chain per molecule, including at least one fatty chain with a length of less than 16 carbon atoms, and mixtures thereof.

20. Use according to Claim 18 or 19, characterized in that the spherules active at the surface are formed of at least one lipid chosen from:
Sunflower lecithin;
Soya lecithin/ethanol/water;
Soya lecithin/cholesterol/propylene glycol;
Lauryl polyglyceryl-6-cetearyl glycol ether/dimyristyl phosphate.

21. Cosmetic or dermatological composition for topical treatment, characterized in that it comprises a first dispersion of spherules with deep-down action which are capable of diffusing into the deep layers of the skin, these spherules with deep-down action containing a spin trap employed as an electron paramagnetic resonance measurement probe, and being capable of releasing the spin trap into the deep layers of the skin, a second dispersion of spherules active at the surface which are capable of diffusing into the surface layers of the skin, those spherules active at the surface containing a sunscreen agent and being capable of releasing the sunscreen agent into the surface layers of the skin in order to provide surface protection additional to the deep-down protection, and a cosmetically or dermatologically acceptable medium.

22. Composition according to Claim 20, characterized in that the spin trap is chosen from the 2,2,6,6-tetramethylpiperidine 1-oxide, 4-hydroxytetramethylpiperidine 1-oxide, N-tert-butyl-alpha-phenylnitrone and doxylcyclohexane radicals, and the salts of N-(1-oxido-2,2,6,6-tetramethyl-4-piperidyl)-N,N-dimethyl-N-hydroxyethylammonium.

23. Composition according to either of Claims 21 and 22, characterized in that the spin trap is present in an amount of from 0.001 % to 5 % by weight relative to the total weight of the composition.

24. Composition according to any one of Claims 21 to 23, characterized in that the spin trap is present in an amount of from 0.1 % to 2 % by weight relative to the total weight of the composition.

25. Composition according to any one of Claims 21 to 24, characterized in that the spherules with deep-down action are in the gelled state at room temperature.

26. Composition according to any one of Claims 21 to 25, characterized in that the spherules with deep-down action are formed of lipids comprising at least one linear and saturated fatty chain having from 16 to 30 carbon atoms.

27. Composition according to any one of Claims 21 to 26, characterized in that the spherules with deep-down action are formed by lipids chosen from:
Triglyceryl cetyl ether/cholesterol/casein lipoamino acid;
Mixture of triglyceryl mono-, di- and tricetyl ether/cholesterol/dicetyl phosphate;
Triglyceryl cetyl ether/cholesterol/dicetyl phosphate;
Sorbitan palmitate/cholesterol/sodium acylglutamate;
PEG 8 stearate/cholesterol/sodium acylglutamate;
Diglyceryl distearate/cholesterol/sodium acylglutamate;
Sucrose mono- and distearate/cholesterol/sodium acylglutamate;
PEG 8 stearate/cholesterol/phytanetriol/sodium acylglutamate;
Polyoxyethylenated methylglucose distearate containing 20 mol of ethylene oxide/cholesterol/sodium acylglutamate;
Hydrogenated lecithin/polyoxyethylenated phytosterol;
Tetraglyceryl tristearate/cholesterol/sodium acylglutamate.

28. Composition according to any one of Claims 21 to 27, characterized in that the spherules active at the surface are formed of lipids chosen from natural ionic phospholipids containing unsaturated fatty chains having from 16 to 30 carbon atoms, alkyl ethers or alkyl eaters having at least one fatty chain per molecule, including at least one fatty chain with a length of less than 16 carbon atoms, and mixtures thereof.

29. Composition according to any one of Claims 21 to 28, characterized in that the spherules active at the surface are formed of at least one lipid chosen from: Sunflower lecithin;
Soya lecithin/ethanol/water;
Soya lecithin/cholesterol/propylene glycol;
Lauryl polyglyceryl-6-cetearyl glycol ether/dimyristyl phosphate.

30. Composition according to any one of Claims 21 to 29, characterized in that it is provided in the form of a gel, an emulsion, a lotion, a serum or oil droplets dispersed by lipid vesicles.

31. Composition according to any one of Claims 21 to 30, characterized in that it additionally contains an oily phase which is dispersed in an aqueous phase.

32. Composition according to any one of Claims 21 to 31, characterized in that it additionally contains hydrophilic adjuvants.

33. Composition according to any one of Claims 21 to 32, characterized in that the spherules additionally contain active agents chosen from moisturizing agents, keratolytic agents, antimicrobial agents and anti-inflammatory agents, vitamins, protides, sugars, trace elements, enzymes and essential oils.

34. Process for the light-protective, anti-ageing and/or anti-acne cosmetic treatment of the skin, consisting in applying to, the skin a composition according to any one of Claims 21 to 33.

35. Light-protective, anti-ageing and/or anti-acne cosmetic treatment process which consists in applying to the skin of the human face or body a cosmetic or dermatological composition comprising a first dispersion of spherules with deep-down action which are capable of diffusing into the deep layers of the skin, these spherules containing a spin trap employed as an electron paramagnetic resonance measurement probe, and a cosmetically or dermatologically acceptable medium.

36. Cosmetic or dermatological composition comprising a first dispersion of spherules with deep-down action which are capable of diffusing into the deep layers of the skin, these spherules containing a spin trap employed as an electron paramagnetic resonance measurement probe, and a cosmetically or dermatologically acceptable medium, this composition being in the form of an emulsion or droplets of oil dispersed by lipid vesicles.

## Patentansprüche

1. Kosmetische Verwendung von Spin-Akzeptoren, die als Meßsonden bei der elektronenparamagnetischen Resonanz eingesetzt werden, in kosmetischen oder dermatologischen Zusammensetzungen zur Lichtschutzbehandlung der Haut und/oder zur Behandlung der Haut gegen Alterung und/oder gegen Akne.

2. Verwendung von Spin-Akzeptoren, die als Meßsonden bei der elektronenparamagnetischen Resonanz eingesetzt werden, zur Herstellung dermatologischer Zusammensetzungen zur Lichtschutzbehandlung der Haut und/oder zur Behandlung der Haut gegen Alterung und/oder gegen Akne.

3. Verwendung nach Anspruch 1 oder 2, dadurch gekennzeichnet, daß der Spin-Akzeptor unter stabilen Nitroxyl-Radikalen und cyclischen Nitronen, die mindestens einen Ring aufweisen, der ein an ein Sauerstoff gebundenes Stickstoffatom enthält, ausgewählt ist.

4. Verwendung nach Anspruch 3, dadurch gekennzeichnet, daß der Spin-Akzeptor Alkylgruppen in α-Stellung zum Stickstoffatom aufweist.

5. Verwendung nach einem der vorhergehenden Ansprüche, dadurch gekennzeichnet, daß der Spin-Akzeptor unter den Radikalen 2,2,6,6-Tetramethylpiperidin-1-oxyl,
4-Hydroxytetramethylpiperidin-1-oxyl, N-*tert*.-Butyl-α-phenylnitron, Doxylcyclohexan und den N-(1-Oxyl-2,2,6,6-tetramethylpiperidin-4-yl)-N-dimethyl-N- hydroxyethylammoniumsalzen ausgewählt ist.

6. Verwendung nach einem der vorhergehenden Ansprüche, dadurch gekennzeichnet, daß der Spin-Akzeptor in einem Mengenanteil von 0,001 bis 5 Gew.-%, bezogen auf das Gesamtgewicht der Zusammensetzung, vorliegt.

7. Verwendung nach einem der vorhergehenden Ansprüche, dadurch gekennzeichnet, daß der Spin-Akzeptor in einem Mengenanteil von 0,1 bis 2 Gew.-%, bezogen auf das Gesamtgewicht der Zusammensetzung, vorliegt.

8. Verwendung nach einem der vorhergehenden Ansprüche, dadurch gekennzeichnet, daß der Spin-Akzeptor in Kügelchen eingekapselt ist.

9. Verwendung nach Anspruch 8, dadurch gekennzeichnet, daß die Kügelchen Lipidvesikel sind.

10. Verwendung nach Anspruch 8 oder 9, dadurch gekennzeichnet, daß die Kügelchen Kügelchen mit Tiefenwirkung umfassen, die befähigt sind, in die unteren Hautschichten zu diffundieren.

11. Verwendung nach Anspruch 10, dadurch gekennzeichnet, daß die Kügelchen mit Tiefenwirkung bei Umgebungstemperatur im Gelzustand vorliegen.

12. Verwendung nach Anspruch 10 oder 11, dadurch gekennzeichnet, daß die Kügelchen mit Tiefenwirkung aus Lipiden bestehen, die mindestens eine geradkettige und gesättigte Fettkette mit 16 bis 30 Kohlenstoffatomen aufweisen.

13. Verwendung nach einem der Ansprüche 10 bis 12, dadurch gekennzeichnet, daß die Kügelchen mit Tiefenwirkung aus Lipiden bestehen, die ausgewählt sind unter:
Triglycerylcetylether/Cholesterin/Casein-Lipoaminosäure;
Gemisch von Triglycerylmono-, -di- und -tricetylether/Cholesterin/Dicetylphosphat;
Triglycerylcetylether/Cholesterin/Dicetylphosphat;
Sorbitanpalmitat/Cholesterin/Natriumacylglutamat;
PEG-8-Stearat/Cholesterin/Natriumacylglutamat;
Diglyceryldistearet/Cholesterin/Natriumacylglutamet;
Saccharosemono- und -distearat/Cholesterin/Natriumacylglutamat;
PEG-8-Stearat/Cholesterin/Phytantriol/Natriumacylglutamat;
polyethoxyliertes Methylglucosedistearat mit 20 mol Ethylenoxid/Cholesterin/Natriumacylglutamat;
hydriertes Lecithin/polyethoxyliertes Phytosterin;
Tetraglyceryltristearat/Cholesterin/Natriumacyl
glutamat.

14. Verwendung nach einem der vorhergehenden Ansprüche, dadurch gekennzeichnet, daß die Zusammensetzung in Form eines Gels, einer Emulsion, einer Lotion, eines Serums oder von Öltröpfchen vorliegt, die durch Lipidvesikel dispergiert sind.

15. Verwendung nach einem der vorhergehenden Ansprüche, dadurch gekennzeichnet, daß die Zusammensetzung ferner eine in einer wäßrigen Phase dispergierte Ölphase enthält.

16. Verwendung nach einem der vorhergehenden Ansprüche, dadurch gekennzeichnet, daß die Zusammensetzung ferner hydrophile Hilfsstoffe enthält.

17. Verwendung nach einem der vorhergehenden Ansprüche, dadurch gekennzeichnet, daß die Zusammensetzung ein Sonnenschutzfilter enthält.

18. Verwendung nach Anspruch 17, dadurch gekennzeichnet, daß das Sonnenschutzfilter in Kügelchen für die Hautoberfläche eingekapselt ist, die befähigt sind, in die oberen Hautschichten einzudiffundieren.

19. Verwendung nach Anspruch 18, dadurch gekennzeichnet, daß die Kügelchen mit Oberflächenwirkung aus Lipiden bestehen, die ausgewählt sind unter natürlichen ionischen Phospholipiden mit ungesättigten Fettketten mit 16 bis 30 Kohlenstoffatomen und Alkylethern oder Alkylestern von Polyolen mit mindestens einer Fettkette pro Molekül, wobei mindestens eine Fettkette eine Länge von weniger als 16 Kohlenstoffatomen aufweist, sowie deren Gemischen.

20. Verwendung nach Anspruch 18 oder 19, dadurch gekennzeichnet, daß die Kügelchen mit Oberflächenwirksamkeit aus mindestens einem Lipid bestehen, das ausgewählt ist unter:
Sonnenblumenlecithin;
Sojalecithin/Ethanol/Wasser;
Sojalecithin/Cholesterin/Propylenglykol;
Laurylpolyglyceryl-6-cetearylglykolether/Dimyristylphosphat.

21. Kosmetische oder dermatologische Zusammensetzung zur topischen Behandlung, dadurch gekennzeichnet, daß sie enthält: eine erste Dispersion von Kügelchen mit Tiefenwirkung, die befähigt sind, in die unteren Hautschichten einzudiffundieren, und einen Spin-Akzeptor enthalten, der als Meßsonde bei der elektronenparamagnetischen Resonanz eingesetzt wird, und in der Lage sind, den Spin-Akzeptor in den unteren Hautschichten freizusetzen, eine zweite Dispersion von Kügelchen mit Oberflächenwirkung, die befähigt sind, in die oberflächlichen Hautschichten einzudiffundieren, und ein Sonnenschutzfilter enthalten und in der Lage sind, das Sonnenschutzfilter in den oberflächlichen Hautschichten freizusezten, um einen Oberflächenschutz zu gewährleisten, der den Tiefenschutz ergänzt, sowie ein kosmetisch oder dermatologisch akzeptables Medium.

22. Zusammensetzung nach Anspruch 20, dadurch gekennzeichnet, daß der Spin-Akzeptor unter den Radikalen 2,2,6,6-Tetramethylpiperidin-1-oxyl,
4-Hydroxytetramethylpiperidin-1-oxyl, N-*tert*.-Butyl-α-phenylnitron, Doxylcyclohexan und den N-(1-Oxyl-2,2,6,6-tetramethylpiperidin-4-yl)-N-dimethyl-N-hydroxyethylammoniumsalzen ausgewählt ist.

23. Zusammensetzung nach Anspruch 21 oder 22, dadurch gekennzeichnet, daß der Spin-Akzeptor in einem Mengenanteil von 0,001 bis 5 Gew.-%, bezogen auf das Gesamtgewicht der Zusammensetzung, vorliegt.

24. Zusammensetzung nach einem der Ansprüche 21 bis 23, dadurch gekennzeichnet, daß der Spin-Akzeptor in einem Mengenanteil von 0,1 bis 2 Gew.-%, bezogen auf das Gesamtgewicht der Zusammensetzung, vorliegt.

25. Zusammensetzung nach einem der Ansprüche 21 bis 24, dadurch gekennzeichnet, daß die Kügelchen mit Tiefenwirkung bei Umgebungstemperatur im Gelzustand vorliegen.

26. Zusammensetzung nach einem der Ansprüche 21 bis 25, dadurch gekennzeichnet, daß die Kügelchen mit Tiefenwirkung aus Lipiden bestehen, die mindestens eine geradkettige und gesättigte Fettkette mit 16 bis 30 Kohlenstoffatomen aufweisen.

27. Zusammensetzung nach einem der Ansprüche 21 bis 26, dadurch gekennzeichnet, daß die Kügelchen mit Tiefenwirkung aus Lipiden bestehen, die ausgewählt sind unter:
Triglycerylcetylether/Cholesterin/Casein-Lipoaminosäure;
Gemisch von Triglycerylmono-, -di- und -tricetylether/Cholesterin/Dicetylphosphat;
Triglycerylcetylether/Cholesterin/Dicetylphosphat;
Sorbitanpalmitat/Cholesterin/Natriumacylglutamat;
PEG-8-Stearat/Cholesterin/Natriumacylglutamat;
Diglyceryldistearat/Cholesterin/Natriumacylglutamat;
Saccharosemono- und -distearat/Cholesterin/Natriumacylglutamat;
PEG-8-Stearat/Cholesterin/Phytantriol/Natriumacylglutamat;
polyethoxyliertes Methylglucosedistearat mit 20 mol Ethylenoxid/Cholesterin/Natriumacylglutamat;
hydriertes Lecithin/polyethoxyliertes Phytosterin;
Tetraglyceryltristearat/Cholesterin/Natriumacylglutamat.

28. Zusammensetzung nach einem der Ansprüche 21 bis 27, dadurch gekennzeichnet, daß die Kügelchen mit Oberflächenwirkung aus Lipiden bestehen, die ausgewählt sind unter natürlichen ionischen Phospholipiden mit ungesättigten Fettketten mit 16 bis 30 Kohlenstoffatomen und Alkylethern oder Alkylestern von Polyolen mit mindestens einer Fettkette pro Molekül, wobei mindestens eine Fettkette eine Länge von weniger als 16 Kohlenstoffatomen aufweist, sowie deren Gemischen.

29. Zusammensetzung nach einem der Ansprüche 21 bis 28, dadurch gekennzeichnet, daß die Kügelchen mit Oberflächenwirksamkeit aus mindestens einem Lipid bestehen, das ausgewählt ist unter:
Sonnenblumenlecithin;
Sojalecithin/Ethanol/Wasser;
Sojalecithin/Cholesterin/Propylenglykol;
Laurylpolyglyceryl-6-cetearylglykolether/Dimyristylphosphat.

30. Zusammensetzung nach einem der Ansprüche 21 bis 29, dadurch gekennzeichnet, daß sie in Form eines Gels, einer Emulsion, einer Lotion, eines Serums oder von Öltröpfchen, die durch Lipidvesikel dispergiert sind, vorliegt.

31. Zusammensetzung nach einem der Ansprüche 21 bis 30, dadurch gekennzeichnet, daß sie ferner eine in einer wäßrigen Phase dispergierte Ölphase enthält.

32. Zusammensetzung nach einem der Ansprüche 21 bis 31, dadurch gekennzeichnet, daß sie ferner hydrophile Hilfsstoffe enthält.

33. Zusammensetzung nach einem der Ansprüche 21 bis 32, dadurch gekennzeichnet, daß die Kügelchen ferner Wirkstoffe enthalten, die ausgewählt sind unter Hydratisierungsmitteln, Keratolytika, antimikrobiellen und entzündungshemmenden Mitteln, Vitaminen, Protiden, Zuckern, Spurenelementen, Enzymen und etherischen Ölen.

34. Verfahren zur kosmetischen Behandlung der Haut zur Erzielung eines Lichtschutzes, gegen Alterung und/oder gegen Akne, das darin besteht, daß eine Zusammensetzung nach einem der Ansprüche 21 bis 33 auf die Haut aufgetragen wird.

35. Verfahren zur kosmetischen Behandlung zur Erzielung eines Lichtschutzes, gegen Alterung und/oder gegen Akne, das darin besteht, daß eine kosmetische oder dermatologische Zusammensetzung, die eine erste Dispersion von Kügelchen mit Tiefenwirkung enthält, die befähigt sind, in die unteren Hautschichten einzudiffundieren, und einen Spin-Akzeptor enthalten, der als Meßsonde bei der elektronenparamagnetischen Resonanz eingesetzt wird, sowie ein kosmetisch oder dermatologisch akzaptables Medium enthält, auf die Haut des Gesichts oder des menschlichen Körpers aufgetragen wird.

36. Kosmetische oder dermatologische Zusammensetzung, die eine erste Dispersion von Kügelchen mit Tiefenwirkung, die befähigt sind, in die unteren Hautschichten einzudiffundieren, und einen Spin-Akzeptor enthalten, der als Meßsonde bei der elektronenparamagnetischen Resonanz eingesetzt wird, sowie ein kosmetisch oder dermatologisch akzaptabies Medium enthält, wobei die Zusammensetzung in Form einer Emulsion oder von Öltröpfchen, die durch Lipidvesikel dispergiert sind, vorliegt.
